(19) **Europäisches Patentamt / European Patent Office / Office européen des brevets**

(11) **EP 2 182 891 B1**

(12) **EUROPEAN PATENT SPECIFICATION**

(45) Date of publication and mention
of the grant of the patent:
**09.04.2014 Bulletin 2014/15**

(51) Int Cl.:
***A61F 2/16*** (2006.01)

(21) Application number: **08798477.9**

(86) International application number:
**PCT/US2008/073999**

(22) Date of filing: **22.08.2008**

(87) International publication number:
**WO 2009/029515 (05.03.2009 Gazette 2009/10)**

(54) **MULTIZONAL ASPHERIC LENS WITH EXTENDED DEPTH OF FOCUS**

ASPHÄRISCHE MEHRZONENLINSE MIT VERGRÖSSERTER FOKUSTIEFE

LENTILLE ASPHÉRIQUE À ZONES MULTIPLES COMPORTANT UNE PROFONDEUR DE FOYER ACCRUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.08.2007 US 845682**

(43) Date of publication of application:
**12.05.2010 Bulletin 2010/19**

(73) Proprietor: **Abbott Medical Optics Inc.**
**Santa Ana, CA 92705-4933 (US)**

(72) Inventor: **BOGAERT, Theophilus**
**NL-9745DX Groningen (NL)**

(74) Representative: **HOFFMANN EITLE**
**Patent- und Rechtsanwälte**
**Arabellastrasse 4**
**81925 München (DE)**

(56) References cited:
| | |
|---|---|
| EP-A- 1 424 049 | EP-A1- 0 926 531 |
| WO-A-01/63344 | WO-A-01/82839 |
| WO-A-02/21194 | WO-A-2004/090611 |
| WO-A-2005/019906 | WO-A-2006/060477 |
| WO-A1-2004/034129 | US-A- 4 637 697 |
| US-A- 6 126 286 | US-A1- 2006 030 938 |
| US-A1- 2006 116 765 | US-B2- 6 609 793 |

- LIOU L H ET AL: "Anatomically accurate, finite model eye for optical modeling", JOURNAL OF THE OPTICAL SOCIETY OF AMERICA A, OPTICAL SOCIETY OF AMERICA, US, vol. 14, no. 8, 1 August 1997 (1997-08-01) , pages 1684-1695, XP008082210, ISSN: 1084-7529
- H Liou ET AL: "The prediction of spherical aberration with schematic eyes", ophthalmologic & physiological optics, vol. 16, no. 4, 4 November 2002 (2002-11-04), pages 348-354, XP55007665,

## Description

Field of the Invention

This invention relates to devices and method for enhancing the vision of a subject and, more particularly, to multi-zonal ophthalmic lenses and method of making that correct aberrations and provide an extended depth of focus.

Background of the Invention

Intraocular lenses and other ophthalmic devices are used to restore and correct vision. For example, monofocal intraocular lenses may be used to replace the natural lens of an eye that has developed cataracts. The simplest types of lenses to fabricate are generally spherical lenses in which both surfaces of the lens have a spherical profile. More recently, aspheric lenses have been used to replace or supplement the eye's natural lens. Such aspheric lenses may be used to at least partially correct for aberrations that are produced by spherical surfaces and/or aberrations produced by the eye itself (e.g., positive spherical aberrations produced by the cornea of most human eyes). Examples of such lens designs are described in USPN 6,609,793 and 7,137,702. Lenses may also be configured to correct for chromatic aberrations inherent in most refractive lenses, for example, through the use of diffractive phase plates (e.g., USPN 4,637,697, 5,121,979, and 6,830,332 and U.S. Patent Application Number 2004/0156014 and 2006/0098163.

When spherical intraocular lenses are used, a practitioner may select a lens power based on a so called "hyperfocal distance", which may make a subject slightly myopic. One advantage of this choice is an increased likelihood that the subject will have spectacle free vision for at least one distance (e.g., if preoperative measurements result in an intraocular lens that is too strong, then the lens will at least provide near or intermediate vision without the use of spectacles or contact lenses). Another benefit of this approach is that lens power selection based on the hyperfocal distance generally provides for the greatest range of distances over which objects at different distances will be reasonably clear to the subject, without the use of spectacles or contact lenses. The increased focal range provided by choosing the hyperfocal distance results in a type of pseudo-accommodation that can resemble the vision provided by the eye's natural lens prior to the onset of presbyopia.

An intraocular lens with increasing then decreasing power is known from WO 02/21194.

A phakic intraocular lens for pre-presbyopes is mentioned in US 2006/0116765.

One potential drawback to selecting the optical power of an intraocular lens to correspond to the hyperfocal distance is that visual acuity for nighttime driving may be reduced, since the best lens performance has been set for objects located between the hyperfocal distance and optical infinity. By contrast, most of the objects within the field of view under these conditions are at optical infinity and, therefore, slightly defocused. Since the pupil is fully dilated under these conditions, spherical aberrations may further reduce visual acuity. Spherical aberrations may be reduced by using aspheric lens surfaces that are configured to correct or compensate for spherical aberrations of the lens and/or cornea.

Regardless of in-focus condition selected (e.g., at optical infinity or at the hyperfocal distance), aspheric lens surfaces serve to provide an improved visual outcome. This is because, as compared to a substantially equivalent spherical lens, aspheric lenses generally provide better visual acuity or MTF performance at all distances and not simply at the distance corresponding to the in-focus condition wherein light is focused on the surface of the retina. Thus, while an aspheric lens with a power selected for the hyperfocal distance generally provides better nighttime driving vision than is possible with a spherical lens, the visual acuity will still be at least somewhat reduced as compared that when the power of the lens is selected to provide emmetropia.

Accordingly, improved designs in monofocal ophthalmic lenses are needed that will provide both increased visual acuity under nighttime driving conditions, while also providing the relatively large depth of focus under other lighting conditions that is possible by selecting a lens power based on the hyperfocal distance.

US 6,126,286 discloses an intraocular lens having nine optical zones. Vision correction power alternates between the zones from a progressive and continuous decrease to a progressive and continuous increase.

Summary of the Invention

Embodiments of the present invention as defined in claim 1 are generally directed to devices and methods for providing an eye with enhanced visual acuity under certain visual and/or lighting conditions (e.g., by reducing spherical aberrations or other aberrations of the lens and/or eye under typical nighttime driving conditions) while simultaneously providing a relatively large depth of field or depth of focus under other lighting conditions as compared to traditional spherical and/or aspheric lenses (e.g., under typical indoor or outdoor lighting conditions or under typical reading conditions). Exemplary embodiments of the invention presented herein are directed to intraocular lenses.

One aspect of the present invention involves an ophthalmic device, as defined in claim 1. such as an intraocular lens, comprising an optic having a variation in optical power over the entire clear aperture that is less than a predetermined amount that is relatively small compared to the add power of a typical refractive or diffractive multifocal intraocular lens, less than 1.5 Diopters. The optic has a clear aperture over which incident light is focused onto the retina of an eye, an anterior surface, and an opposing posterior surface, the surfaces disposed about an optical axis. The optic further comprises a central zone having a plurality of optical powers that progressively vary between a first optical power at a center of the central zone and a second optical power at a periphery of the central zone, wherein the absolute value of the difference between the first optical power and the second optical power is within a relatively small range compared to the add power of a typical refractive or diffractive multifocal intraocular lens, between about 0.5 Diopter and about 1.5 Diopters. The ophthalmic devices also comprises an outer zone disposed about the central zone, the outer zone comprising a third optical power and optionally an optical aberration to compensate or reduce a similar aberration of the cornea or eye of a subject. The optical aberration may be a chromatic aberration or a monochromatic aberration such as a spherical aberration, a coma, or an astigmatism. Further features of the intraocular lens are defined in claim 1. Another aspect of the present invention involves a method of making an intraocular lens or other ophthalmic device, the method comprising forming an anterior surface and an opposing posterior surface, the surfaces being disposed about an optical axis and providing a clear aperture. The method also comprises forming a central zone comprising a plurality of optical powers that progressively vary between a first optical power at a center of the central zone and a second optical power at a periphery of the central zone. The method further comprises forming outer zone disposed about the central zone, the outer zone comprising a third optical power and an optionally an optical aberration. The optic has a variation in optical power over the entire clear aperture that is less than a predetermined amount that is relatively small compared to the add power of atypical multifocal intraocular lens. Further features of the method of the present invention are defined in claim 10.

Brief Description of the Drawings

Embodiments of the present invention may be better understood from the following detailed description when read in conjunction with the accompanying drawings and as defined in claim 1. The drawings include the following figures:

FIG. 1 is a side view of an intraocular lens comprising spherical surfaces and disposed within an eye.
FIG. 2 is a side view of the intraocular lens of FIG. 1 in which an object or point source is disposed nearer the eye. FIG. 3 is a side view of an intraocular lens comprising at least one aspheric surface configured to reduce a spherical aberration of the eye.
FIG. 4A is a side view of an intraocular lens comprising a central zone and a peripheral zone according to an embodiment of the present invention.
FIG. 4B is a front view of the intraocular lens of FIG. 4A. FIG. 5 is a magnified side view of the intraocular lens of FIG. 4A particularly showing the central zone of the intraocular lens.
FIG. 6 is a is another central zone for use in an intraocular lens, which is not according to an embodiment of the present invention.

Detailed Description of the Drawings

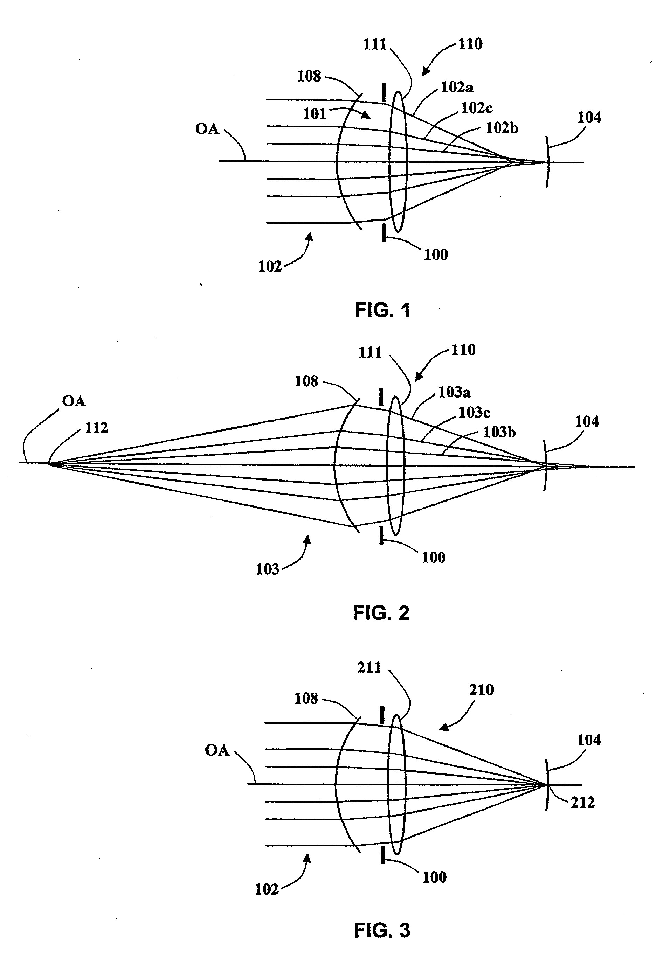

Referring to FIG. 1, an eye is generally disposed about an optical axis OA and comprises an iris 100 forming a pupil 101 through which a plurality of rays 102 from a distant object or point source enter the pupil 101 and are generally focused onto a retina 104 by a cornea 108 and a monofocal intraocular lens 110 comprising an spherical optic 1 1 1 with anterior and posterior surfaces that are spherical. For simplicity, other portions and elements of the eye apart from those shown in FIG. 1 have been left out. The combination of the spherical surfaces of the intraocular lens 110 and the corneal surface shape cause peripheral ray 102a to focus closer to the intraocular lens 110 than does inner or paraxial ray 102b. The local length of the intraocular lens 110 may be represented by an intermediate ray 102c that focuses on the optical axis OA at a location intermediate to the foci of the rays 102a, b. The ray 102c may correspond to a ray that intercepts the optical axis OA at a plane located at the so-called "circle of least" confusion, although other criteria may be used for determining and defining the focal length of the intraocular lens 110. For purposes of this disclosure, the focal length of an intraocular lens (or a region or zone thereof) is the reciprocal of an average optical power over the intraocular lens (or region or zone), where the optical power is expressed in units of Diopters or $m^{-1}$.
The difference in focal location of the rays 102a-c illustrated in FIG. 1 is due, at least in part, to a variation in optical power of the optic 111 with radius from the optical axis OA that results from the use of anterior and posterior lens surfaces that are spherical. This difference in focal location is referred to as a spherical aberration. Since the peripheral ray 102a comes to focus closer to the optic 111 than the paraxial ray 102b, the spherical aberration is said to be a positive spherical aberration. A negative spherical aberration would occur if the peripheral ray 102a were to come to focus farther from the optic 111 than the paraxial ray 102b. The spherical aberration illustrated in FIG. 1 is the result of the combined spherical aberrations optic 111 and the cornea 108.
When the optical power of an optic or optical system varies continuously with distance from the optical axis OA (e.g., as illustrated in FIG. 1), the range of resulting foci along the optical axis OA may be related to an increased depth of focus or depth of field (DOF) of the optic. In the case of the intraocular lens 110, the optical power of the lens is selected so that the paraxial ray 102b is focused onto the retina 104. Thus, at least some light from the distant point source represented in FIG. 1 is focused onto the retina 104 so that the intraocular lens 110 provides distant vision that is clinically equivalent to, or at least similar to, that provided if the intraocular lens 110 were configured to focus the ray 102c onto the retina 104.

With further reference to FIG. 2, an advantage of the arrangement illustrated in FIG. 1 is that an object or point source 112 located at a so-called hyperfocal distance from the eye is also just focused by the optic 111 and cornea 108. As illustrated, a plurality of rays 103 from object source 112 are focused by the cornea 108 and the intraocular lens 110 (e.g., rays 103a-c). The peripheral ray 103a is just focused onto the retina 104, so that an object or point source disposed at the hyperfocal distance is perceived with similar visual acuity as a distant point source at optical infinity. Such an arrangement allows the spherical intraocular lens 110 and/or cornea 108 to provide a pseudo-accommodation in which both distant objects and objects located at intermediate distances (e.g., at or near the hyperfocal distance) are suitably resolved by the eye. As used herein, the term "hyperfocal distance" means a distance from a healthy, emmetropic eye, at which an add power of 0.5 Diopters in the spectacle plane provides visual acuity at least 20/20, based on the standard Snellen test for visual acuity. For example, in a human eye with an axial length (AL) of 25 mm, the hyperfocal distance is approximately 2.5 meters from the eye. As used herein, the term "emmetropic eye" means an eye having a visual acuity for distant vision of at least 20/20, based on the standard Snellen test for visual acuity. As used herein, the term "emmetropic vision" means vision which provides a visual acuity for distant object of at least 20/20.

Referring now to FIG. 3, an aspheric intraocular lens 210 is illustrated that comprises an optic 211 in which at least one of the surfaces is aspheric, such that all the rays 102 from a distant object or point source come to a common focus 212 on the surface of the retina 104. The surfaces of the optic 211 may be configured such that the optic 211 has a negative spherical aberration that is selected to compensate, reduce, or cancel a positive spherical aberration produced by a spherical surface of the optic 211 and/or by a spherical aberration of the cornea 108. Additionally or alternatively, the intraocular lens 210 may be configured to comprise another monochromatic aberration that compensates, reduces, or cancels a substantially opposite aberration of the cornea 108 and/or a remaining spherical surface of the optic 211. As discussed in USPN 6,609,793, the cornea 108 may represent an average model cornea (e.g., based on a population having a common characteristic). In such instances, the surfaces of the optic 211 may have a negative spherical aberration that partially or completely compensates for a spherical aberration of the cornea 108. The population may, for example, represent patients that are candidates for a cataract surgery, patients within a certain age group, and/or having a common or similar aberration or set of aberrations.

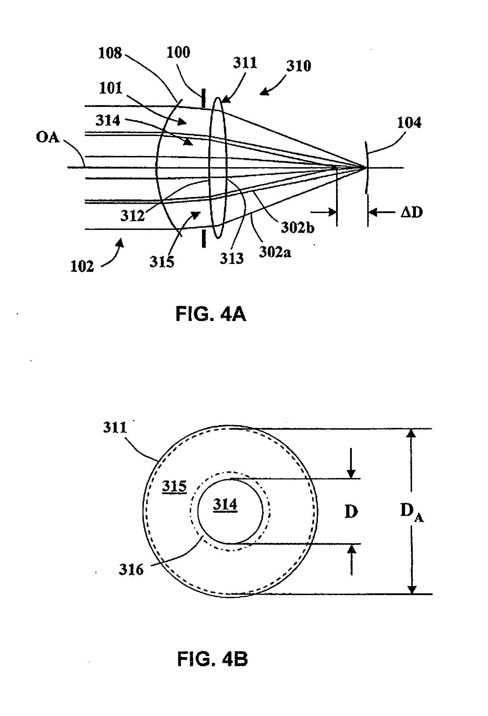

Referring to FIGS. 4A and 4B, an intraocular lens 310 according to an embodiment of the present invention is disposed within an eye and comprises an optic 311. The optic 311 is configured to provide an extended DOF (e.g., similar to that illustrated for the intraocular lens 110) under certain lighting conditions, while also providing enhanced visual acuity (e.g., similar to that produced by an aspheric intraocular lens illustrated for the intraocular lens 210) for other lighting conditions. The optic 311 has a clear aperture over which light incident thereon is focus onto the retina 104. The optic 311 includes an anterior surface 312 and an opposing posterior surface 313, the surfaces 312, 313 being disposed about an optical axis OA.

As used herein, the term "clear aperture" means the opening of a lens or optic that restricts the extent of a bundle of light rays from a distant source that can imaged or focused by the lens or optic. The clear aperture is usually circular and specified by its diameter. Thus, the clear aperture represents the full extent of the lens or optic usable in forming the conjugate image of an object or in focusing light from a distant point source to a single focus or to a plurality of predetermined foci, in the case of a multifocal optic or lens. It will be appreciated that the term "clear aperture" does not denote or imply a particular clarity or transmissivity of an optic or lens. For example, an optic may have a clear aperture that is approximately equal to the diameter of the optic, irrespective of whether or not a dye is used to reduce the transmission of light.

In the illustrated embodiment, the clear aperture has a diameter DA that is substantially equal to the diameter of the optic 311, As illustrated in FIG. 4B, the diameter DA may be slightly smaller than the outer diameter of the optic 311, for example, due to the presence of a peripheral edges that includes rounded corners not useful in focusing light or forming an image on the retina 104. In some embodiment, the peripheral edge is configured to reduce scatter from light sources at the periphery of the field of view of a subject into which the lens 210 is implanted. Thus, the glare reducing peripheral edge may slightly reduces the clear aperture of the optic 311.

The optic 311 comprises a central zone 314 and an outer zone 315 disposed about the central zone 314. The central zone 314 includes a plurality of optical powers that progressively vary between a first optical power PI at a center of the central zone 314 and a second optical power P2 at a periphery of the central zone 314. The absolute value of the difference between the first optical power P1 and the second optical power P2 (e.g., |P2-P1|) is less than the add power of a typical multifocal intraocular lens (e.g., less than about 3 or 4 Diopters). The absolute difference between P1 and P2 is between about 0.5 Diopter and about 1.5 Diopter.

The outer zone 315 of optic 311 comprises a third optical power P3 that may be equal to P1, between P1 and P2, or outside the range between P1 and P2. Either or both of the zones 314, 315 may include a monochromatic and/or chromatic aberration that is selected to improve vision when the pupil 101 is relatively large (e.g., under low light conditions or typical room light). For example, at least one of the surfaces 312, 313 in the vicinity of the outer zone 315 may have a negative spherical aberration that at least partially compensates for a positive spherical aberration of the cornea and/or for a positive spherical aberration of one or both of the surfaces of the optic 31 1. The outer zone has an outer diameter that is equal to the outer diameter of the clear aperture.

The zones 314, 315 are configured such that the optic 311 has a variation in optical power over the entire clear aperture that is less than about 1.5 Diopters or less that about 1 Diopter. The total variation in optical power over the entire clear aperture may be selected in accordance with specific design parameters such as the range of pseudo-accommodation to be provided, the required visual acuity at one or more specific object distance, the zone diameters, the pupil size under certain lighting conditions, the expected variation in pupil size, a desired mixture of near, intermediate, and/or distant vision for one or more pupil sizes, and the like.

The optic 311 in the illustrated embodiment is circular; however, other shapes may be used, for example, to enhance the insertion characteristics of the intraocular lens 310 into the eye through a small incision. Also, at least one of the zones 314, 315 may comprise a cylinder power, for example, to correct for an astigmatism of the eye. While not illustrated in the FIGS. 4A or 4B, it will be appreciated that the intraocular lens 310 may generally comprise other features and elements such as haptics for centering the intraocular lens 310 within the eye, and/or a positioning structure for providing accommodative axial motion and/or deformation of the optic 311. The optic 311 may be a single optic or part of a lens system, for example, one of the lenses of a two lens accommodating intraocular lens. In addition, the intraocular lens 310 may be configured to attenuate light over a band of wavelengths light outside a band of wavelengths. In such embodiments the intraocular lens 310 or the optic 311 may incorporate one or more dyes or other substances or devices for selectively blocking incident radiation, for example, to selectively blocking UV radiation or light in the violet or blue bands of the visible spectrum.

The intraocular lenses 310 may be fabricated with optical powers that vary from about 10 Diopters to about 30 Diopters in increments of about 0.5 Diopters. In some embodiments, intraocular lenses 310 may be produced that vary from about zero Diopters to about 40 Diopters or more. Alternatively or additionally, intraocular lenses 310 may be produced that have a negative optical power, for example that is within a range of less than about zero Diopters to greater than about -20 Diopters or less.

The intraocular lens 310 may generally be constructed of any of the various types of material known in the art. For example, the intraocular lens 310 may be a foldable lens made of at least one of the materials commonly used for resiliently deformable or foldable optics, such as silicone polymeric materials, acrylic polymeric materials, hydrogel-forming polymeric materials (e.g., polyhydroxyethylmethacrylate, polyphosphazenes, polyurethanes, and mixtures thereof), and the like. Other advanced formulations of silicone, acrylic, or mixtures thereof are also anticipated. Selection parameters for suitable lens materials are well known to those of skill in the art. See, for example, David J. Apple, et al., Intraocular Lenses: Evolution, Design, Complications, and Pathology, (1989) William & Wilkins. The lens material may be selected to have a relatively high refractive index, and thus provide a relatively thin optic, for example, having a center thickness in the range of about 150 microns to about 1000 microns, depending on the material and the optical power of the lens. At least portions of the intraocular lens 310, for example one or more haptics or fixation members thereof, may be constructed of a more rigid material including such polymeric materials as polypropylene, polymethylmethacrylate PMMA, polycarbonates, polyamides, polyimides, polyacrylates, 2-hydroxymethylmethacrylate, poly (vinylidene fluoride), poiytetrafluoroethylene and the like; and metals such as stainless steel, platinum, titanium, tantalum, shapememory alloys, e.g., nitinol, and the like. In some embodiments, the optic and haptic portions of the intraocular lens 310 are integrally formed of a single common material.

As illustrated in FIG. 4A, a pair of rays 302a, 302b from the light 102 impinge upon the outer zone 315 near the outer periphery thereof and near the central zone 314, respectively. At least one of the surfaces 312, 313 in the region of the outer zone 315 is preferably aspheric in shape, such that light passing through the outer zone 315 is focused to substantially a single point or focus (e.g., to within a circle about the size of an Airy disk defining a diffraction limit of the zone 315). For example, the outer zone 315 may be configured to have at least some of the features and/or functions describe above with regards to the optic 211 illustrated in FIG. 3. In this regard, the outer zone 315 may comprise a monochromatic aberration, such as a spherical aberration, that corrects or at least partially compensates for an aberration of the eye (e.g., a spherical aberration introduced by the cornea 108). Additionally or alternatively, the outer zone 315 may incorporate a chromatic aberration, for example, through the use of a diffractive grating or phase plate on one of the lens surfaces. The aberration of the outer zone 315 may be selected to correct the aberrations of an individual cornea, in which case the intraocular lens 310 may be a custom intraocular lens. Alternatively, the intraocular lens 310 may be selected from a plurality intraocular lenses or optic portions having the same optical power, but differing amounts of spherical aberration. Alternatively, the aberration of the outer zone 315 may be selected to compensate for an aberration of a cornea that is part of an eye model and/or that represents an average cornea based on a particular population (e.g., an average spherical aberration for a population of people of a particular age group or that are likely candidates of a particular surgical procedure). The outer zone 315 of the intraocular lens 310 may be configured to have a surface sag profile that varies according to the relation:

$$\frac{cr^2}{1+\sqrt{1-(1+k)c^2r^2}}+a_4r^4+a_6r^6+\dots$$

wherein $a_2 \dots a_l$ ,... are constants, c is a base curvature of the surface portion, $k$ is a conic constant, and r is the radial distance from the optical axis OA.

The aberration of the outer zone 315 may be selected to completely or substantially completely compensate for a spherical aberration of a cornea or eye. Alternatively, the aberration of the outer zone 315 may be selected to only partially compensate for (or over compensate for) the spherical aberration or other aberration of the cornea or eye. In this regard, it may be advantageous in certain embodiments to select the aberration of the outer zone 315 to leave a residual aberration when combined with a cornea, for example, as discussed in U.S. Patent Application Number 2006/0244904. For instance, the intraocular lens 310 may comprise an outer zone 315 that has an optical power that is about 20 Diopters and a negative spherical aberration that partially correct a positive spherical aberration of the cornea, wherein the outer zone 315 has a negative spherical aberration that is between about -0.19 and about -0.202 microns, or that is about -0.156 microns.

The eye may have a residual aberration that is essentially zero or is greater than zero (e.g., a residual aberration of about +0.14 microns or between about +0.006 microns and about +0.090 microns has been reported as potentially beneficial, for example, when placed in an eye or an eye model with a corneal spherical aberration of about 0.327 microns). In other embodiments, the intraocular lens 310 is configured with an outer zone 315 in which the optical power at the periphery of the zone is about 0.5 to about 0.75 Diopters less than the optical power at or near the boarder between the zones 314, 315.

As discussed above, the central zone 314 has an optical power that ranges from P1 at the center to P2 at the periphery of the zone, while the outer zone 315 has a power P3. In certain embodiments, the first optical power P1 and/or the third optical power P3 is less than the second optical power P2 by an amount that is less than about 1.5 Diopters, preferably less than or equal to about 1.0 Diopter, and in some cases or equal to about 0.5 Diopters. In some embodiments, the variation in optical power over the entire clear aperture (e.g., within and between the zones 314, 315) is less than or equal to about 0.5 Diopter plus the variation in optical power produced by the spherical aberrations of a spherical optic having a nominal optical power equal to that of the third optical power P3.

In the illustrated embodiment, the difference between the second optical power P2 of the central zone 314 and the first and/or third optical powers P1, P3 represents an add power $\Delta D$, where the add power $\Delta D$ is generally smaller than the add power of a typical multifocal intraocular lens, which generally have add powers in the range of about 2 Diopters to about 4 Diopters (see, for example, USPN's 6,527,389, 5,225,858, and 6,557,992. As used herein, the term "add power" means a change in optical power from an optical power necessary to provide distance vision. As used herein, the "add power" is the change in power at the principal plane of the intraocular lens (e.g., an intraocular lens add power of 4.0 Diopters is approximately equal to an increase in optical power of about 3.2 Diopters in the spectacle lens). Surprisingly, the use of a relatively small add power according to embodiments of the invention may beneficially provide better intermediate vision and/or near vision than if a larger add power were to be used in the central zone 314 (e.g., an add power of about 3 or 4 Diopters). This improved performance may, for example, be due to relatively low noise from halo effects when using a lower add power.

Embodiments of the intraocular lens 310 may be configured to provide a pupil 101 dependent visual acuity performance that is preferred over either a spherical intraocular lens such as the intraocular lens 110 or an aspheric intraocular lens such as the intraocular lens 210. For example, both zones 314, 315 in the illustrated embodiment focus light onto or near the retina 104 when the pupil 101 is relatively large, for instance under low lighting conditions or night time driving conditions. Because at least one of the surfaces 312, 313 in the vicinity of the outer zone 315 is aspheric, most of the light from distant objects entering the optic 311 is advantageously focused to substantially a single focus or point. This may provide better visual acuity than is generally possible with an optic having only spherical surfaces (e.g., the intraocular lens 110 illustrated in FIG. 1). The relative areas of the zones 314, 315 may be selected to provide more light energy for distant vision under lower lighting conditions. Thus, while some light from distant object entering the central zone 314 may be slightly defocused, relatively high visual acuity may be maintained, since most of the light entering the optic 311 under these conditions is focused by the outer zone 315 onto the surface of the retina 104

The intraocular lens 310 is also able to provide a pseudo-accommodative benefits under bright or intermediate lighting conditions in which the pupil 101 is small, since under these conditions all or most of the light entering the intraocular lens 310 passes through the central zone 314. Thus, the intraocular lens 310 is able to advantageously provide pseudo-accommodative benefits without significantly compromising the advantages of an aspheric intraocular lens over a spherical intraocular lens during night driving conditions.

The performance of the intraocular lens 310 under differing pupil sizes may be controlled by selecting the diameter of the central zone 314. For example, the central zone 314 may be configured to have an outer diameter D that is about the size of a typical pupil that is fully contracted, such as under sunny outdoor lighting conditions (e.g., the diameter D of the central zone 314 may about 1 millimeter, about 2 millimeters, or about 3 millimeters, or between about 2 millimeters and about 3 millimeters, depending on the relative performance desired between near, intermediate, and distant vision). In other embodiments, the diameter D is selected to provide predetermined areas ratios of the central and outer zones 314, 315 under specific lighting conditions or pupil sizes. Thus, the diameter D may be selected to provide a predetermined performance balance of distant visual acuity and enhanced DOF (or pseudo-accommodation) as a function of pupil size.

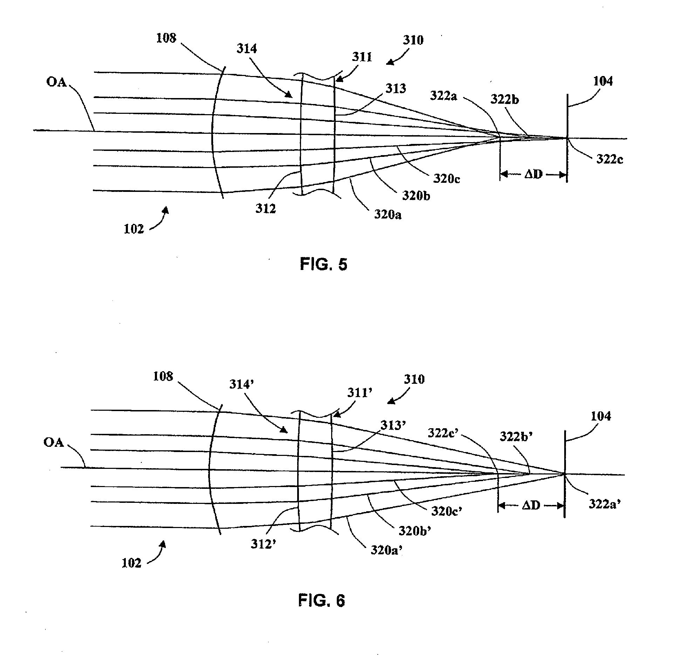

To illustrate one way of configuring the central zone 314 to provide pseudo-accommodation, reference is now made to FIG. 5, which is a magnified side view of the central zone 314 of the optic 311. Three rays 320a, 320b, 320c are shown intercepting the central zone 314 at different radial distances from the optical axis OA, the peripheral ray 320a intercepts a peripheral region of the central zone 314 and is focused along the optical axis OA to a focus 322a, while the paraxial ray 320c intercepts the central zone 314 at or near the optical axis OA and is focused along the optical axis OA to a focus 322c. The intermediate ray 320b intercepts the central zone 314 at a location between the rays 320a, 320c and is focused along the optical axis OA to a focus 322c. In this case the peripheral ray 320a represents a maximum optical power (the first optical power P1) of the central zone 314. The focal length (or optical power) of the central zone 314 may be represented by the distance between the focal point 322b of the intermediate ray 320b and a principle plane of the central zone 314 or the optic 311. Alternatively, the focal length of the central zone 314 may be represented by another point between the foci 322a, 322c.

As illustrated in FIG. 5, the central zone 314 comprises a plurality of optical powers that progressively and continuously increases from the first optical power P1 (e.g., represented by the focus 322c) to the second optical power P2 (e.g., represented by the focus 322a) as the radius from the optical axis OA increases. In such embodiments, the third optical power P3 of the outer zone 315 may be selected to be equal that of first optical power P1 of the central zone 314.

As discussed in greater detail below, the central zone 314 may alternatively, but such an alternative is not an embodiment of the invention, comprise plurality of optical powers that progressively and continuously decreases from a first optical power P1 (e.g., represented by the focus 322c' in FIG. 6) to the second optical power P2 (e.g., represented by the focus 322a' in FIG. 6) as the radius from the optical axis OA increases. In any event, the optic 311 is generally configured to provided enhanced visual acuity (e.g., with the outer zone 315) for distant vision and at least reasonably good visual acuity at intermediate distances under certain conditions (e.g., with the central zone 313), In one embodiment, the third optical power P3 is selected to provide distant vision when the intraocular lens is disposed within the eye, while the first and second optical powers P1, P2 are selected so that the central zone 314 provides a visual acuity of at least 20/30, or even 20/20, for objects located at a hyperfocal distance from the eye.

The paraxial ray 320c comes to focus on the retina 104 at the focus 322c, so that objects a optical infinity are just focused and, therefore, at least somewhat resolved by the eye. Referring to the discussion above with regards to FIG. 2, the central zone 314 may be similarly constructed to the optic 21 1 (e.g., comprising anterior and posterior surfaces that are spherical) so that objects located at the hyperfocal distance advantageously cause the peripheral ray 320a to be focused on the retina 104 and, therefore, to provide a visual acuity of least 20/40, 20/30, or even 20/20. Thus, the central zone 314 in the illustrated embodiment has an extended DOF (represented by the distance $\Delta D$) that provides pseudo-accommodation, by allowing objects located at distances between optical infinity and the hyperfocal distance to resolved by the eye. For object closer than the hyperfocal distance, all the light or rays are generally focused posteriorly to the retina 104, wherein other devices or means may be necessary to provide a desirable level of visual acuity.

In some embodiments, at least one of the surfaces of the central zone 314 has a positive spherical aberration that is greater than that of an equivalent spherical surface having substantially the same focal length or optical power. In general the amount of positive spherical aberration may be selected to provide a predetermined DOF and/or add power, as represented by $\Delta D$ in FIG. 5. For example, at least one of the surfaces 312, 313 in the vicinity of the central zone 314 may be an oblate surface that has a greater curvature in the periphery than in the center. In such embodiments, the central zone 314 may be configured to suitably resolve objects that are closer than the hyperfocal distance and/or provide enhanced intermediate vision. As used herein the term "intermediate vision" means vision of objects situated approximately 40 centimeters to approximately 1.5 meters from the eye or spectacle plane. By contrast, the term "near vision" means to vision produced by an eye that allows a subject to focus on objects or planes that are relatively close to the subject, for example, within a range of about 25-40 cm or at a distance at which a subject would generally place printed material for the purpose of reading. As used herein, the term "distant vision" means vision produced by an eye that allows a subject to focus on objects or planes that are relatively distant from the subject, preferably at a distance that is greater than about 1 meter to about 2 meters away from the subject, more preferably at a distance of 5 to 6 meters away or greater.

Referring to FIG. 6, which does not form an embodiment of the invention, the optic 311 may comprise a central zone 314' that has a negative spherical aberration. For example, in the illustrated embodiment, at least one of the surface 312', 313' has a negative spherical aberration that may be selected to produce an overall negative spherical aberration when placed in the eye of a subject. In effect, the optical power of central zone 314' generally decreases with increasing radial distance from the optical axis OA and may be configured such that the third optical power P3 of the outer zone 315 is equal or substantially equal that of the optical power P2 of the central zone 314. Thus, a peripheral ray 320a' of the central zone 314' is focused at or near the retina 104, while intermediate and paraxial rays 320b', 320c' are focused progressively closer to the central zone 314'. The resulting add power ∆D may be represented by the change in focal length over the central zone 314' (e.g., between the foci 322a' and 322c').

In certain embodiments, a method of making an intraocular lens comprises forming an anterior surface and an opposing posterior surface, the surfaces being disposed about an optical axis to provide a clear aperture. The method further comprises forming a central zone comprising a plurality of optical powers that progressively vary between a first optical power at a center of the central zone and a second optical power at a periphery of the central zone, the absolute value of the difference between the first optical power and the second optical power being between about 0.5 Diopter and about 1 Diopter, The method also comprises forming outer zone disposed about the central zone, the outer zone comprising a third optical power and a negative spherical aberration. The optic resulting from the method has a variation in optical power over the entire clear aperture that is less than about 1 Diopter.

**Claims**

1.   An intraocular lens, comprising:

    an optic comprising:

        a clear aperture having a diameter;
        an anterior surface and an opposing posterior surface, the surfaces disposed about an optical axis;
        a central zone having a plurality of optical powers that progressively vary between a first optical power at a center of the central zone and a second optical power at a periphery of the central zone,
        the absolute value of the difference between the first optical power and the second optical power being between about 0.5 Diopter and about 1.5 Diopters, wherein the plurality of optical powers progressively and continuously increase from the first optical power to the second optical power, wherein the central zone has a diameter that is between about 1 millimeter and about 3 millimeters; and
        an outer zone disposed about the central zone, the outer zone comprising a third optical power and a negative spherical aberration over the entire zone, the outer zone having an outer diameter that is equal to the diameter of the clear aperture,
        wherein there is a border between the outer zone and the central zone;

    the optic having a variation in optical power over the entire clear aperture that is less than about 1.5 Diopters.

2.   The intraocular lens of claim 1, wherein the absolute value of the difference between the first optical power and the second optical power is about one Diopter.

3.   The intraocular lens of claim 1, wherein the first optical power is equal to the third optical power.

4.   The intraocular lens of any of claim 1, wherein at least one of the zones has a cylinder power.

5.   The intraocular lens of claim 1, wherein
    the absolute value of the difference between the first optical power and the second optical power is between about 0.5 Diopter and about 1 Diopter; and
    the optic has a variation in optical power over the entire clear aperture that is less than about 1 Diopter.

6.   The intraocular lens of claim 1, wherein the third optical power is equal to the first optical power or between the first optical power and the second optical power.

7.   The intraocular lens of claim 1 having optical powers that vary from about 10 Diopters to about 30 Diopters.

8.   The intraocular lens of claim 1, wherein the outer zone is configured so that the optical power at the periphery of the outer zone is about 0.5 to about 0.75 Diopters less than the optical power at the border between the central zone and the outer zone.

9.   The intraocular lens of claim 1, wherein the diameter of the central zone is about 1 mm, about 2 mm or about 3 mm or between about 2 mm and 3 mm.

10.  A method of making an intraocular lens of claim 1, comprising:

forming an anterior surface and an opposing posterior surface, the surfaces being disposed about an optical axis and providing a clear aperture;

forming a central zone comprising a plurality of optical powers that progressively and continuously increase from a first optical power at a center of the central zone to a second optical power at a periphery of the central zone, the absolute value of the difference between the first optical power and the second optical power being between about 0.5 Diopter and about 1 Diopter, wherein the central zone has a diameter that is between about 1 mm and about 3 mm;

forming an outer zone disposed about the central zone, the outer zone comprising a third optical power and a negative spherical aberration over the entire zone,

wherein the outer zone has a border with the central zone and has an outer diameter that is equal to the diameter of the clear aperture;

the optic having a variation in optical power over the entire clear aperture that is less than about 1 Diopter.

## Patentansprüche

1. Intraokulare Linse, aufweisend:

eine Optik, mit:

einer freien Apertur, die einen Durchmesser aufweist;

einer anterioren Fläche und einer gegenüberliegenden posterioren Fläche, wobei die Flächen um eine optische Achse angeordnet sind;

einer mittigen Zone, die eine Vielzahl optischer Stärken aufweist, die sich progressiv zwischen einer ersten optischen Stärke in einer Mitte der mittigen Zone und einer zweiten optischen Stärke in einem Randbereich der mittigen Zone ändert, wobei der Absolutwert der Differenz zwischen der ersten optischen Stärke und der zweiten optischen Stärke zwischen in etwa 0,5 Dioptrie und in etwa 1,5 Dioptrien ist, die Vielzahl optischer Stärken progressiv und fortlaufend von der ersten optischen Stärke zu der zweiten optischen Stärke ansteigen und die mittige Zone einen Durchmesser aufweist, der zwischen in etwa 1 mm und in etwa 3 mm ist; und

einer äußeren Zone, die um die mittige Zone angeordnet ist, wobei die äußere Zone eine dritte optische Stärke und eine negative sphärische Aberration über die gesamte Zone aufweist und die äußere Zone einen Außendurchmesser aufweist, der dem Durchmesser der freien Apertur gleicht, wobei zwischen der äußeren Zone und der mittigen Zone eine Begrenzung vorhanden ist;

wobei die Optik eine Änderung der optischen Stärke über die gesamte freie Apertur aufweist, die geringer als in etwa 1,5 Dioptrien ist.

2. Intraokulare Linse nach Anspruch 1, bei welcher der absolute Differenzwert zwischen der ersten optischen Stärke und der zweiten optischen Stärke in etwa ein Dioptrie ist.

3. Intraokulare Linse nach Anspruch 1, bei der die erste optische Stärke der dritten optischen Stärke gleicht.

4. Intraokulare Linse nach Anspruch 1, bei der zumindest eine der Zonen eine zylindrische Stärke aufweist.

5. Intraokulare Linse nach Anspruch 1, bei welcher der Absolutwert der Differenz zwischen der ersten optischen Stärke und der zweiten optischen Stärke zwischen in etwa 0,5 Dioptrie und in etwa 1 Dioptrie ist; und die Optik eine Änderung der optischen Stärke über die gesamte freie Apertur aufweist, die geringer als 1 Dioptrie ist.

6. Intraokulare Linse nach Anspruch 1, bei der die dritte optische Stärke der ersten optischen Stärke gleicht oder zwischen der ersten optischen Stärke und der zweiten optischen Stärke ist.

7. Intraokulare Linse nach Anspruch 1, die optische Stärken aufweist, die von in etwa 10 Dioptrien bis in etwa 30 Dioptrien variieren.

8. Intraokulare Linse nach Anspruch 1, bei der die äußere Zone so eingerichtet ist, dass die optische Stärke in dem Randbereich der äußeren Zone in etwa 0,5 bis in etwa 0,75 Dioptrie geringer ist, als die optische Stärke in dem Grenzbereich zwischen der mittigen Zone und der äußeren Zone.

9. Intraokulare Linse nach Anspruch 1, bei welcher der Durchmesser der mittigen Zone in etwa 1 mm, in etwa 2 mm oder in etwa 3 mm oder zwischen in etwa 2 mm und 3 mm ist.

10. Verfahren zum Herstellen einer Intraokularlinse, umfassend:

Ausbilden einer anterioren Fläche und einer gegenüberliegenden posterioren Fläche, wobei die Flächen um eine optische Achse angeordnet sind und eine freie Apertur bereitstellen;

Ausbilden einer mittigen Zone mit einer Vielzahl optischer Stärken, die progressiv und fortlaufend von einer ersten optischen Stärke in einer Mitte der mittigen Zone zu einer zweiten optischen Stärke in einem Randbereich der mittigen Zone ansteigen, wobei der Absolutwert der Differenz zwischen der ersten optischen Stärke und der zweiten optischen Stärke zwischen in etwa 0,5 Dioptrie und in etwa 1 Dioptrie ist und die mittige Zone einen Durchmesser aufweist, der zwischen in etwa 1 mm und in etwa 3 mm ist; Ausbilden einer äußeren Zone, die um die mittige Zone angeordnet ist, wobei die äußere Zone eine dritte optische Stärke und eine negative sphärische Aberration über die gesamte Zone aufweist und die äußere Zone einen Grenzbereich mit der mittigen Zone aufweist und die äußere Zone einen Außendurchmesser aufweist, der dem Durchmesser der freien Apertur gleicht; wobei die Optik eine Änderung der optischen Stärke über die gesamte freie Apertur aufweist, die geringer als 1 Dioptrie ist.

## Revendications

1. Lentille intra-oculaire, comprenant :

    une optique comprenant :

      une ouverture utile ayant un diamètre ;
      une surface antérieure et une surface postérieure lui faisant face, les surfaces étant disposées par rapport à un axe optique ;
      une zone centrale ayant une pluralité de puissances optiques qui varient progressivement entre une première puissance optique au centre de la zone centrale et une deuxième puissance optique à la périphérie de la zone centrale, la valeur absolue de la différence entre la première puissance optique et la deuxième puissance optique se situant entre environ 0,5 dioptrie et environ 1,5 dioptrie, la pluralité des puissances optiques augmentant de manière progressive et continue depuis la première puissance optique jusqu'à la deuxième puissance optique, la zone centrale ayant un diamètre qui est compris entre environ 1 millimètre et environ 3 millimètres ; et
      une zone externe disposée autour de la zone centrale, la zone externe comprenant une troisième puissance optique et une aberration sphérique négative sur toute la zone, la zone externe ayant un diamètre externe qui est égal au diamètre de l'ouverture utile, un bord étant prévu entre la zone externe et la zone centrale ;

    l'optique présentant une variation de puissance optique sur toute l'ouverture utile qui est inférieure à environ 1,5 dioptrie.

2. Lentille intra-oculaire selon la revendication 1, dans laquelle la valeur absolue de la différence entre la première puissance optique et la deuxième puissance optique est d'environ une dioptrie.

3. Lentille intra-oculaire selon la revendication 1, dans laquelle la première puissance optique est égale à la troisième puissance optique.

4. Lentille intra-oculaire selon la revendication 1, dans laquelle au moins l'une des zones a la puissance d'un cylindre.

5. Lentille intra-oculaire selon la revendication 1, dans laquelle :

      la valeur absolue de la différence entre la première puissance optique et la deuxième puissance optique se situe entre environ 0,5 dioptrie et environ 1 dioptrie ; et
      l'optique présente une variation de puissance optique sur toute l'ouverture utile qui est inférieure à environ 1 dioptrie.

6. Lentille intra-oculaire selon la revendication 1, dans laquelle la troisième puissance optique est égale à la première puissance optique, ou se situe entre la première puissance optique et la deuxième puissance optique.

7. Lentille intra-oculaire selon la revendication 1, ayant des puissances optiques qui varient depuis environ 10 dioptries environ jusqu'à environ 30 dioptries.

8. Lentille intra-oculaire selon la revendication 1, dans laquelle la zone externe est configurée de telle manière que la puissance optique à la périphérie de la zone externe est inférieure d'environ 0,5 à environ 0,75 dioptrie à la puissance optique au niveau du bord situé entre la zone centrale et la zone externe.

9. Lentille intra-oculaire selon la revendication 1, dans laquelle le diamètre de la zone centrale est d'environ 1 mm, d'environ 2 mm ou d'environ 3, ou bien se situe entre environ 2 mm et 3mm.

10. Procédé de réalisation d'une lentille intra-oculaire, consistant à :

      former une surface antérieure et une surface postérieure lui faisant face, les surfaces étant disposées par rapport à un axe optique et fournissant une ouverture utile ;
      former une zone centrale comprenant une pluralité de puissances optiques qui varient de manière progressive et continue entre une première puissance optique au centre de la zone centrale et une deuxième puissance optique à la périphérie de la zone centrale, la valeur absolue de la différence entre la première puissance optique et la deuxième puissance optique se situant entre environ 0,5 dioptrie et environ 1 dioptrie, la zone centrale ayant un diamètre qui est compris entre environ 1 millimètre et environ 3 millimètres ;

former une zone externe disposée autour de la zone centrale, la zone externe comprenant une troisième puissance optique et une aberration sphérique négative sur toute la zone, la zone externe présentant un bord par rapport à la zone centrale et ayant un diamètre externe qui est égal au diamètre de l'ouverture utile ;

l'optique présentant une variation de puissance optique sur toute l'ouverture utile qui est inférieure à environ 1 dioptrie.

**FIG. 1**

**FIG. 2**

**FIG. 3**

**FIG. 4A**

**FIG. 4B**

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040156014 A
- US 20060098163 A
- WO 0221194 A
- US 20060116765 A
- US 6126286 A
- US 20060244904 A

**Non-patent literature cited in the description**

- **DAVID J. APPLE et al.** Intraocular Lenses: Evolution, Design, Complications, and Pathology. William & Wilkins, 1989